(19) Europäisches Patentamt
European Patent Office
Office européen des brevets

(11) Numéro de publication: **0 319 028 B1**

(12) # FASCICULE DE BREVET EUROPEEN

(45) Date de publication de fascicule du brevet: **19.08.92** (51) Int. Cl.5: **A61K 7/06**

(21) Numéro de dépôt: **88120162.8**

(22) Date de dépôt: **02.12.88**

Le dossier contient des informations techniques présentées postérieurement au dépôt de la demande et ne figurant pas dans le présent fascicule.

(54) **Association de dérivés de 1, 8-hydroxy et/ou acyloxy anthracène ou anthrone et de dérivés de pyrimidine pour induire et stimuler la croissance des cheveux et diminuer leur chute.**

(30) Priorité: **04.12.87 LU 87066**

(43) Date de publication de la demande:
**07.06.89 Bulletin 89/23**

(45) Mention de la délivrance du brevet:
**19.08.92 Bulletin 92/34**

(84) Etats contractants désignés:
**AT BE CH DE FR GB IT LI NL SE**

(56) Documents cités:
**GB-A- 2 085 442**
**GB-A- 2 184 721**
**US-A- 4 139 619**

(73) Titulaire: **L'OREAL**
**14, Rue Royale**
**F-75008 Paris(FR)**

(72) Inventeur: **Grollier, Jean Francois**
**16 bis Boulevard Morland**
**F-75004 Paris(FR)**

(74) Mandataire: **Casalonga, Alain et al**
**BUREAU D.A. CASALONGA - JOSSE Morassistrasse 8**
**W-8000 München 5(DE)**

**EP 0 319 028 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention est relative à l'association de dérivés de 1,8-hydroxy et/ou acyloxy anthracène ou anthrone et de dérivés de pyrimidine en vue d'induire et de stimuler la croissance des cheveux et diminuer leur chute.

L'homme a un capital de 100.000 à 150.000 cheveux et il est normal de perdre quotidiennement 50 à 100 cheveux. La maintenance de ce capital résulte essentiellement du fait que la vie d'un cheveu est soumise à un cycle dit "cycle pilaire" au cours duquel le cheveu se forme, croît et tombe avant d'être remplacé par un nouveau cheveu qui apparaît dans le même follicule.

On observe au cours d'un cycle pilaire successivement trois phases, à savoir : la phase anagène, la phase catagène et la phase télogène.

Au cours de la première phase, dite anagène, le cheveu passe par une période de croissance active associée avec une intense activité métabolique au niveau du bulbe.

La deuxième phase, dite catagène, est transitoire et elle est marquée par un ralentissement des activités mitotiques. Au cours de cette phase, le cheveu subit une involution, le follicule s'atrophie et son implantation dermique apparaît de plus en plus haute.

La phase terminale, dite télogène, correspond à une période de repos du follicule et le cheveu finit par tomber, poussé par un cheveu anagène naissant.

Ce processus de renouvellement physique permanent subit une évolution naturelle au cours du vieillissement, les cheveux deviennent plus fins et leurs cycles plus courts.

L'alopécie survient lorsque ce processus de renouvellement physique est accéléré ou perturbé, c'est-à-dire lorsque les phases de croissance sont raccourcies, le passage des cheveux en phase télogène est plus précoce et les cheveux tombent en plus grand nombre. Les cycles de croissance successifs aboutissent à des cheveux de plus en plus fins et de plus en plus courts, se transformant peu à peu en un duvet non pigmenté qui peut conduire à la calvitie.

Parmi les alopécies, les alopécies acquises , non cicatricielles et diffuses, telle que l'alopécie androgénétique ou androgénique, sont particulièrement difficiles à traiter par opposition aux alopécies localisées telle que la pelade qui serait d'origine immunologique.

On a déjà proposé, dans le passé, le traitement de la pelade avec l'anthraline ou 1,8,9-trihydroxy anthracène, en produisant une dermatite inflammatoire non-allergique, par contre son utilisation dans le domaine de l'alopécie androgénétique ne s'est pas révélée satisfaisante.

La demande de brevet GB-A-2 184 721 décrit l'utilisation des mono-, di- et triesters de dihydroxy-1,8 phényl-10 anthrone-9 ou anthranol-9 dans le traitement des pellicules, de la séborrhé et de la chute des cheveux.

Par ailleurs, on a également proposé d'utiliser des composés tels que l'amino-6 dihydro-1,2 hydroxy-1 imino-2 pipéridino-4 pyrimidine ou "Minoxidil" dans des compositions permettant de réduire ou de supprimer l'effet de l'alopécie et d'induire et de stimuler la croissance des cheveux et diminuer leur chute.

La demanderesse a découvert maintenant, qu'en associant des dérivés de 1,8-hydroxy et/ou acyloxy anthracène ou anthrone avec certains dérivés de pyrimidine, on constate de façon surprenante une induction et une stimulation améliorées de la croissance des cheveux et une action sur le freinage de la chute des cheveux.

Cette efficacité se caractérise plus particulièrement par une activité supérieure par rapport aux dérivés de pyrimidine ou de 1,8-hydroxy et/ou acyloxy anthracène ou anthrone utilisés seuls, ou encore par une action plus rapide de l'association au niveau du traitement de la chute des cheveux ou par une utilisation du dérivé de pyrimidine à une concentration plus faible, en particulier dans le cas des alopécies diffuses.

Afin de déterminer l'efficacité ou la rapidité d'action des compositions de traitement de l'alopécie, on utilise généralement le trichogramme et en particulier le phototrichogramme qui permet de déterminer entre autre le pourcentage de cheveux en phase anagène par rapport aux cheveux en phase télogène.

Un objet de l'invention est donc constitué par l'association de dérivés de 1,8-hydroxy et/ou acyloxy anthracène ou anthrone avec des dérivés de pyrimidine en vue d'induire ou stimuler la croissance des cheveux et diminuer leur chute.

Un autre objet de l'invention est constitué par les compositions cosmétiques et/ou pharmaceutiques contenant ces composés.

L'invention a également pour objet les dispositifs à plusieurs compartiments contenant, dans un compartiment, le dérivé de 1,8-hydroxy et/ou acyloxy anthracène ou anthrone, et l'autre, le dérivé de pyrimidine, en vue d'un mélange tout juste avant l'emploi ou d'applications simultanées, successives ou bien décalées dans le temps.

D'autres objets de l'invention apparaîtront à la lecture de la description et des exemples qui suivent.

L'association conforme à l'invention est essentiellement caractérisée par le fait qu'elle est constituée par :

(a) un composant (A) comprenant, dans un milieu physiologiquement acceptable, au moins un 1,8-dihydroxy, 1,8-diacyloxy ou 1-hydroxy 8-acyloxy anthracène ou anthrone, choisi parmi :

- le 1,8,9-trihydroxy anthracène ou 1,8-dihydroxy 9-anthrone et son dimère,
- la 1,8,1′,8′-tétrahydroxy dianthrone,
- les 1,8-dihydroxy 9-anthrones substitués en position 10 par des groupements de formules :

i) 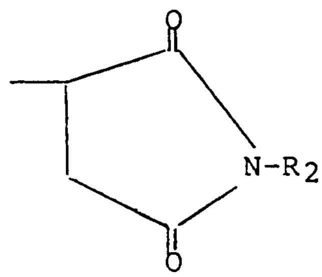

ii) 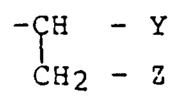

iii) 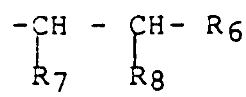

dans lesquelles $R_2$ représente un atome d'hydrogène, un radical alkyle, linéaire ou ramifié, ayant de 1 à 8 atomes de carbone, un radical mono ou polyhydroxyalkyle linéaire ou ramifié, ayant de 1 à 3 atomes de carbone, un radical carbamoyle ou un radical phényle,

Y et Z désignent simultanément un groupement $-CONR_3R_4$ ou bien désignent indépendamment l'un de l'autre, soit un groupement $-CONR_3R_4$ ou soit un groupement $CO_2R_5$, $R_3$, $R_4$ et $R_5$ représentant un atome d'hydrogène, un radical alkyle ayant de 1 à 8 atomes de carbone, un radical mono- ou polyhydroxyalkyle, éventuellement interrompu par un atome d'oxygène ayant de 3 à 8 atomes de carbone, un radical cycloalkyle ayant de 3 à 6 atomes de carbone, ou bien $R_3$ et $R_4$ pris ensemble forment un radical divalent choisi parmi les radicaux suivants :

$-(CH_2)_n-$ où n est égal à 4 ou 5

$-(CH_2)_2-O-(CH_2)_2-$

ou 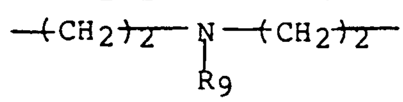

dans laquelle $R_9$ désigne un atome d'hydrogène, un radical méthyle ou hydroxy-2 éthyle.

$R_6$ désigne un groupement $CO_2R_5$, CN, CHO, $CONH_2$ ou bien $CONH-CH_2OH$, dans lequel $R_5$ a la même signification que celle indiquée ci-dessus, $R_7$ et $R_8$ représentent indépendamment l'un de l'autre un atome d'hydrogène ou un radical méthyle ainsi que leurs sels et isomères optiques,

- les adducts de 1,8-dihydroxy 9-anthrone, comportant aux positions 9 et 10 les radicaux divalents suivants :

iv) 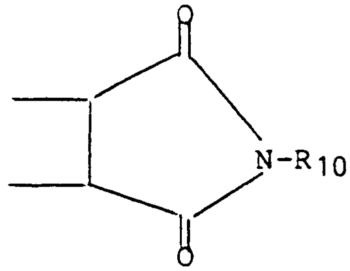

v) 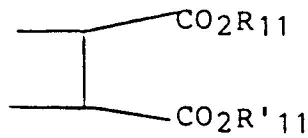

vi) 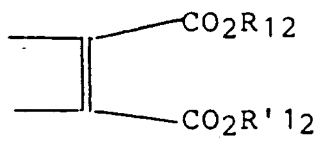

dans laquelle $R_{10}$, $R_{11}$ et $R'_{11}$ représentent un atome d'hydrogène, un radical alkyle inférieur, linéaire ou ramifié, ayant de 1 à 8 atomes de carbone, un radical monohydroxyalkyle éventuellement interrompu par un ou plusieurs atomes d'oxygène ayant de 2 à 8 atomes de carbone, un radical cycloalkyle ayant de 4 à 6 atomes de carbone, un radical phényle ou un radical benzyle, $R_{12}$ et $R'_{12}$, identiques ou différents, représentent un atome d'hydrogène, un radical alkyle inférieur, linéaire ou ramifié, ayant de 1 à 8 atomes de carbone à l'exclusion de $R_{12} = R'_{12} = CH_3$, un radical monohydroxyalkyle, éventuellement interrompu par un ou plusieurs atomes d'oxygène ayant de 2 à 8 atomes de carbone, un radical phényle ou benzyle et les sels de ces composés;

- les 1,8-dihydroxy 9-anthrones substituées en position 10 par les groupements :

vii) 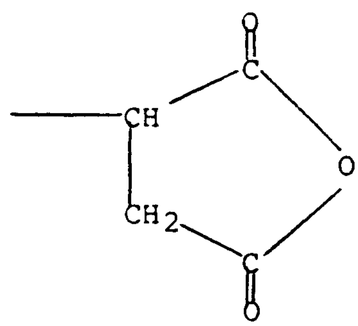

viii) 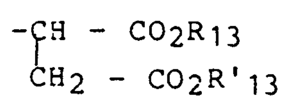

dans lesquels $R_{13}$ et $R'_{13}$, identiques ou différents, représentent un atome d'hydrogène ou un radical méthyle ou éthyle, ou leurs isomères,

ou par le groupement de formule :

ix) 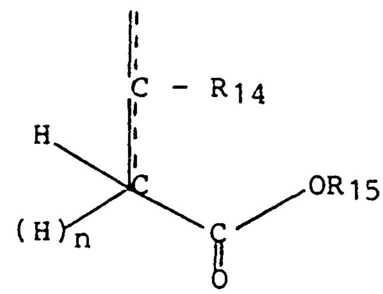

dans laquelle, $R_{14}$ représente un atome d'hydrogène ou le radical $COR'_{15}$, $R_{15}$ et $R'_{15}$, identiques ou différents, représentant un atome d'hydrogène, un radical alkyle, linéaire ou ramifié, ayant de 1 à 6 atomes de carbone, un radical cycloalkyle ayant de 4 à 6 atomes de carbone ou un radical benzyle, ou bien $R_{14}$ et $R_{15}$, pris ensemble, forment un radical

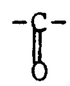

et n est égal à 0 ou 1, ainsi que leurs isomères.

- les aryl-10 dihydroxy-1,8 anthrones dont le groupement aryl répond à la formule suivante :

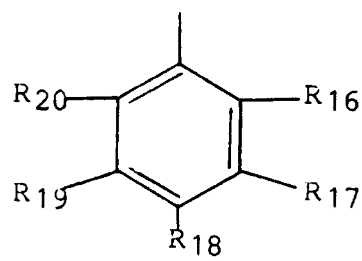

dans laquelle $R_{16}$, $R_{17}$, $R_{18}$, $R_{19}$, $R_{20}$, identiques ou différents, représentent un atome d'hydrogène, un atome d'halogène, le radical $CF_3$, une fonction hydroxyle, un radical alkyle ou cycloalkyle inférieur, un radical hydroxyalkyle inférieur, un radical alcoxy inférieur, une fonction nitrile, un radical

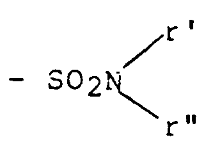 ,

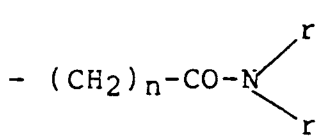 , 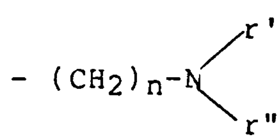 , $-(CH_2)_n-CO_2R'$, ou

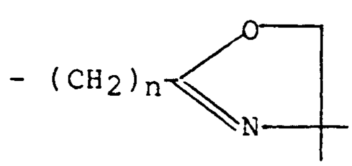 , 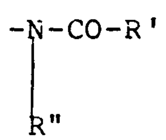

r′, r″, identiques ou différents, représentent un atome d'hydrogène ou un radical alkyle inférieur, n est 0 ou un nombre entier de 1 à 3 inclus, et R′ et R″ représentent un atome d'hydrogène, un radical alkyle inférieur, linéaire ou ramifié, ou un radical aryle éventuellement substitué(s);

- les diacyloxy-1,8 acyl-10 anthrones de formule générale :

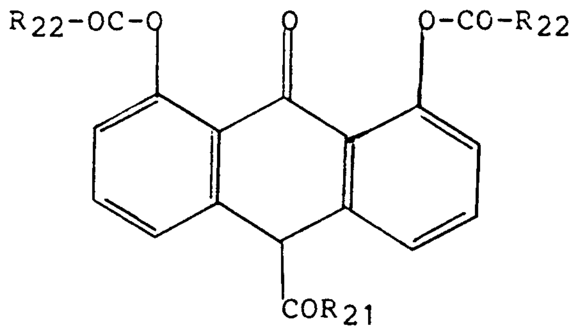

dans laquelle :
$R_{21}$, $R_{22}$, identiques ou différents, représentent un radical alkyle, linéaire ou ramifié, ayant de 1 à 15 atomes de carbone, un radical cycloalkyle de 3 à 6 atomes de carbone, un radical furyl-2 ou -3, un radical pyridyl-3 ou -4, un radical thiényl-2 ou un radical aromatique de formule :

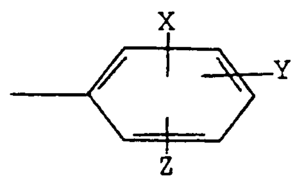

dans laquelle X, Y, Z, identiques ou différents, représentent un atome d'hydrogène, un radical alkyle inférieur, un radical trifluorométhyle, un radical alcoxy inférieur, un atome d'halogène, un groupement nitro ou hydroxyle;

- l'acétyl-10 triacétoxy-1,8,9 anthracène de formule :

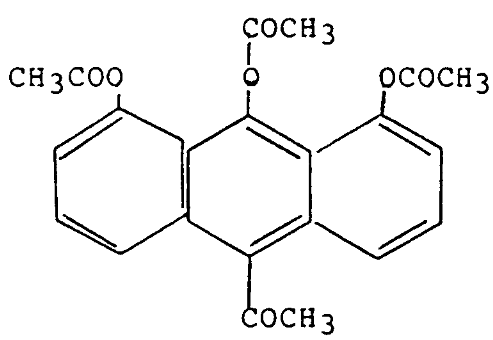

- les hydroxy-1, acyloxy-8 acyl-10 anthrones de formule générale :

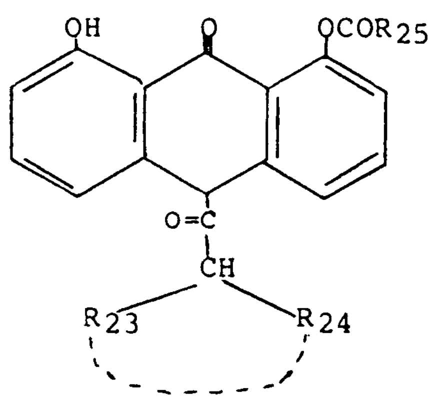

dans laquelle :
$R_{23}$ représente un atome d'hydrogène ou un radical alkyle inférieur linéaire;
$R_{24}$ représente un atome d'hydrogène, un radical alkyle linéaire ou ramifié, ayant de 1 à 17 atomes de carbone, un radical alcényle, linéaire ou ramifié, ayant de 2 à 17 atomes de carbone ou $R_{23}$ et $R_{24}$ pris ensemble forment avec l'atome de carbone auquel ils sont attachés, un radical cycloalkyle ayant de 3 à 6 atomes de carbone.
$R_{25}$ représente un radical alkyle, linéaire ou ramifié, ayant de 1 à 18 atomes de carbone, un radical alkényle, linéaire ou ramifié, ayant de 2 à 18 atomes de carbone, un radical cycloalkyle ayant de 3 à 6 atomes de carbone, un radical furyle-2 ou -3, un radical pyridyle-3 ou -4, un radical thiényl-2 ou un radical aromatique de formule :

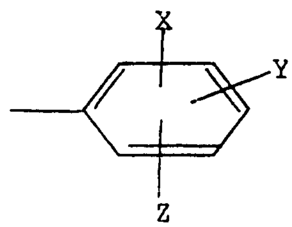

dans laquelle X, Y, Z, identiques ou différents, représentent un atome d'hydrogène, un radical alkyle inférieur, un radical trifluorométhyle, un radical alcoxy inférieur, un atome d'lhalogène, un groupement nitro ou un groupe hydroxyle;

- le triacétoxy-1,8,9 anthracène de formule :

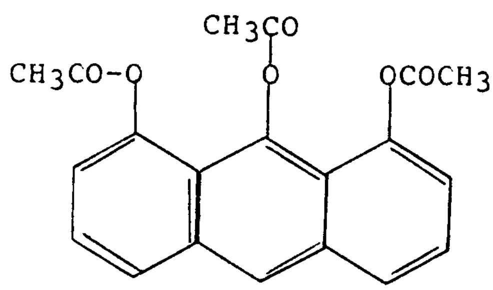

- les mono, di- et tri-esters de dihydroxy-1,8 phényl-10 anthrone-9 ou anthranol-9 de formule générale suivante :

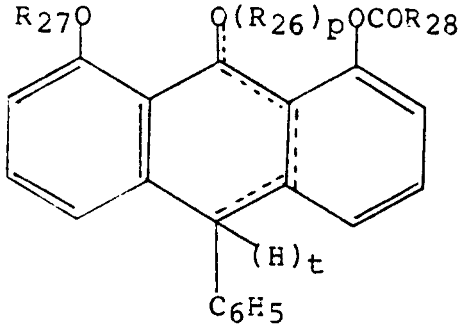

(I)

dans laquelle :
p est 0 ou 1;
. lorsque p = 0, t = 1 et $R_{27}$ représente un atome d'hydrogène ou $-COR_3$;
. lorsque p = 1, t = 0 et $R_{26}$ et $R_{27}$ représentent, indépendamment l'un de l'autre, un atome d'hydrogène ou $-COR_{28}$;

$R_{28}$ représentant un radical alkyle, linéaire ou ramifié, ayant de 1 à 17 atomes de carbone, un radical cycloalkyle, ou un radical phényle éventuellement substitué par un alkyle inférieur, un alcoxy inférieur, un atome d'halogène, une fonction nitro, un radical $-CF_3$ ou par une fonction hydroxyle,

et les mélanges desdits esters.

Ces composés sont décrits plus en détail dans les brevets et demandes de brevets français n°s 2492372, 2492373, 2495934, 2499556, 2565966, 2566772, 2567402, 2567755, 2567756, 2580631, 2591222.

(b) un composant (B) contenant, dans un milieu physiologiquement acceptable, au moins un dérivé de pyyrimidine, répondant à la formule :

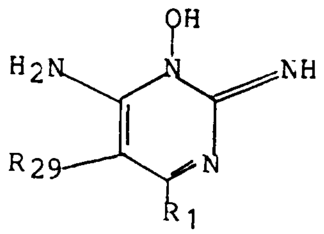

(I)

dans laquelle $R_1$ désigne un groupement

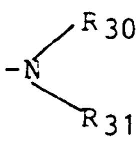

dans lequel $R_{30}$ et $R_{31}$, indépendamment l'un de l'autre, désignent hydrogène, un groupement alkyle, alcényle, alkylaryle, cycloalkyle, $R_{30}$ et $R_{31}$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl(inférieur)-4 pipérazidinyle, les groupements hétérocycliques pouvant être substitués sur les atomes de carbone par un à trois groupements alkyle inférieur, hydroxy ou alcoxy; le groupement $R_{29}$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps, en vue d'induire et stimuler la croissance des cheveux et diminuer leur chute.

Dans la définition des composés utilisés dans l'association conforme à l'invention, le groupement alkyle ou alcoxy désigne de préférence un groupement ayant 1 à 4 atomes de carbone; le groupement alcényle désigne de préférence un groupement ayant 2 à 5 atomes de carbone; le groupement aryle désigne de préférence phényle et cycloalkyle désigne de préférence un groupement ayant 4 à 6 atomes de carbone.

Les composés préférés de formule (I) sont choisis parmi les composés dans lesquels $R_{29}$ désigne hydrogène, et $R_1$ représente un groupement

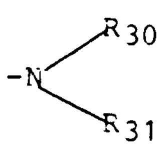

,

dans lequel $R_{30}$ et $R_{31}$ forment un cycle pipéridinyle, ainsi que leurs sels tels que, par exemple, le sulfate.

Parmi ces composés, le composé particulièrement préféré est constitué par l'amino-6 dihydro-1,2 hydroxy-1 imino-2 pipéridino-4 pyrimidine, encore appelé "Minoxidil".

Le dérivé de 1,8-hydroxy et/ou acyloxy anthracène ou anthrone préféré est le 1,8,9-trihydroxy anthracène ou 1,8-dihydroxy 9-anthrone encore connu sous la dénomination d'anthraline ou dithranol.

Le dérivé de 1,8-hydroxy et/ou acyloxy anthracène ou anthrone est utilisé dans le composant (A) dans des proportions comprises entre 0,01 et 10% en poids, et de préférence entre 0,1 et 1% et plus

particulièrement entre 0,1 et 0,5% en poids, le dérivé de pyrimidine de formule (I) est utilisé dans le composant (B) dans des proportions comprises entre 0,05 et 10% en poids, et de préférence entre 0,05 et 5% en poids, et en particulier entre 0,5 et 4% en poids.

Le dérivé de 1,8-hydroxy et/ou acyloxy anthracène ou anthrone est utilisé lorsque les composants (A) et (B) sont dans la même composition, dans des proportions comprises entre 0,01 et 5% en poids par rapport au poids total de la composition, et de préférence entre 0,10 et 1%, et en particulier entre 0,1 et 0,5%. Dans ce cas, le dérivé de pyrimidine de formule (I) est utilisé dans les compositions dans une proportion comprise entre 0,05 et 6% en poids par rapport au poids total de la composition, et de préférence entre 0,1 et 5%, et en particulier entre 0,5 et 4% en poids.

Le rapport pondéral du dérivé de pyrimidine de formule (I) au dérivé de 1,8-hydroxy et/ou acyloxy anthracène ou anthrone est compris, de préférence, entre 2/1 et 10/1.

Le milieu physiologiquement acceptable pour les composants (A) et (B) est un milieu utilisable en pharmacie ou en cosmétique, et peut être constitué par un mélange d'eau et d'un ou plusieurs solvants ou par des solvants essentiellement anhydres, c'est-à-dire contenant moins de 1% d'eau.

Ces compositions peuvent être pressurisées dans des dispositifs aérosols en présence d'un agent propulseur.

Les solvants utilisables sont choisis plus particulièrement parmi les alcools inférieurs en $C_1$-$C_4$,tels que l'alcool éthylique, l'alcool isopropylique, l'alcool tertiobutylique; des alkylèneglycols comme le propylèneglycol, des alkyléthers de mono- et de dialkylèneglycol, tels que plus particulièrement le monoéthyléther d'éthylèneglycol, le monométhyléther de propylèneglycol et le monoéthyléther de diéthylèneglycol.

Les milieux physiologiquement acceptables peuvent être épaissis ou non. Pour épaissir, on peut utiliser des agents épaississants et/ou gélifiants, bien connus dans l'état de la technique. On peut citer plus particulièrement, à cet effet, les hétérobiopolysaccharides tels que la gomme de xanthane ou les scléroglucanes, les dérivés de cellulose, les polymères acryliques réticulés ou non, la poudre de polyéthylène ou de Nylon, des agents épaississants minéraux, tels que la silice colloïdale.

Les solvants, lorsqu'ils sont utilisés, sont présents de préférence dans des proportions comprises entre 1 et 80% en poids, par rapport au poids total de la composition.

Les épaississants peuvent être utilisés, de préférence, dans des proportions comprises entre 0,1 et 5% en poids, et en particulier entre 0,4 et 3% en poids par rapport au poids total de chacun des composants lorsqu'ils sont utilisés de façon séparée ou par rapport au poids total de la composition contenant les composants (A) et (B).

Les compositions constituées, soit par les composants (A) et (B) ou par la composition contenant les deux composants (A) et (B), peuvent également contenir tous autres adjuvants ]habituellement utilisés dans les compositions destinées à une application topique à utilisation cosmétique ou pharmaceutique, et plus particulièrement des agents conservateurs, des agents complexants, des colorants, des agents alcalinisants ou acidifiants, des agents tensio-actifs, anioniques, cationiques, non ioniques, amphotères ou leurs mélanges, des polymères anioniques, cationiques, non ioniques ou amphotères, ainsi que leurs mélanges.

Le pH de ces compositions peut varier entre 4 et 9.

Le composant (A) contenant le dérivé de 1,8-hydroxy et/ou acyloxy anthracène ou anthrone peut être utilisé dans une forme de réalisation particulière avec un milieu permettant d'éviter une dégradation trop brutale du dérivé 1,8-hydroxy et/ou acyloxy anthracène ou anthrone et de le conserver sous sa forme active. A cet effet, on peut utiliser dans le milieu physiologiquement acceptable des agents stabilisants choisis parmi l'acide glycérique, l'acide gluconique, l'acide galacturonique, l'acide malique, l'acide citrique, l'acide tartrique ou l'acide tartronique, dans un véhicule constitué éventuellement de myristate ou de palmitate d'isopropyle ou de monostéarate de glycéryle.

Une autre forme de réalisation peut consister à utiliser comme véhicule unique du dérivé de 1,8-hydroxy et/ou acyloxy anthracène ou anthrone des alkylesters d'acides gras tels que par exemple le myristate d'isopropyle ainsi que des esters aromatiques tel que par exemple le salicylate de benzyle. On peut également utiliser comme véhicule un triglycéride d'acides gras saturés d'origine végétale, ayant de 6 à 18 atomes de carbone, et notamment des triglycérides dont la répartition des acides gras est la suivante :

- acide caproïque (C6) inférieur à 3%
- acide caprylique (C8) 50 à 80%
- acide caprique (C10) 15 à 45%
- acide laurique (C12) inférieur à 5%

et plus particulièrement les produits vendus sous la dénomination MIGLYOL 810 ou MIGLYOL 812 par la Société DYNAMIT NOBEL.

De telles compositions sont décrites plus particulièrement dans les brevets US-4 287 214, 4 367 224, 4 316 902; les demandes de brevets français 2 520 233, 2 515 045, 2 569 561.

Le composant (A) peut également se présenter sous forme d'un gel essentiellement constitué d'huile de paraffine, d'au moins un alkylester d'acide gras et d'une silicone élastomère de la famille des polyvinyldiméthylsiloxanes associés éventuellement à une charge de silice comme décrit dans le brevet français 2 570 602.

Une autre forme de réalisation de l'invention peut consister à utiliser le composant (A) sous forme d'une matrice de diffusion polymérique constituée d'un ester cellulosique et d'un alkylester d'acide gras anhydre comme plastifiant, l'acide gras ayant 5 à 18 atomes de carbone et le radical alkyle linéaire ou ramifié ayant de 3 à 18 atomes de carbone, les esters cellulosiques étant choisis en particulier parmi les acétobutyrates de cellulose et les acétopropionates de cellulose et ayant une viscosité comprise entre 0,0038 et 10 Pa.s

Dans le composant (B), les dérivés de pyrimidine de formule (I) peuvent être présents, soit sous forme dissoute dans le milieu physiologiquement acceptable ou alors, en totalité ou partiellement en suspension dans ce milieu, en particulier sous forme de particules ayant une granulométrie inférieure à 80 $\mu$m et de préférence inférieure à 20 $\mu$m, et en particulier inférieure à 5 $\mu$m.

Une forme de réalisation de l'invention consiste à utiliser l'association conforme à l'invention sous forme d'une composition contenant les composants (A) et (B).

Une forme préférée de réalisation de l'invention, consiste à conserver, dans des dispositifs séparés, les composants (A) et (B) et à préparer la composition contenant le dérivé de 1,8-hydroxy et/ou acyloxy anthracène ou anthrone et le dérivé de pyrimidine (I), de façon extemporanée, tout juste avant application.

Une autre forme de réalisation consiste enfin à appliquer les composants (A) et (B) de façon séparée, soit simultanément, soit de façon successive ou décalée dans le temps.

Dans cette dernière forme de réalisation, on peut, par exemple, appliquer, dans un premier temps, un shampooing contenant le dérivé de 1,8-hydroxy et/ou acyloxy anthracène ou anthrone et plus particulièrement l'anthraline et après avoir laissé agir pendant 5 minutes à 1 heure ce shampooing, on procède à un rinçage et on applique ensuite une lotion à base du dérivé de pyrimidine de formule (I).

Une autre forme de réalisation consiste à appliquer une lotion à base du dérivé de 1,8-hydroxy et/ou acyloxy anthracène ou anthrone, à procéder à un shampooing suivi d'une application d'une autre lotion contenant le dérivé de pyrimidine de formule (I).

L'association conforme à l'invention peut être conditionnée dans ce cas, en particulier, dans un dispositif à plusieurs compartiments appelé "kit" ou nécessaire, dont un premier compartiment contient le composant (A) à base du dérivé de 1,8-hydroxy et/ou acyloxy anthracène ou anthrone et le second compartiment contient le composant (B) à base du dérivé de pyrimidine de formule (I).

Le procédé conforme à l'invention vise le traitement thérapeutique de la chute des cheveux, dans la mesure où il a une action sur le dysfonctionnement des mécanismes biologiques à l'origine de la pousse des cheveux.

Ce procédé peut également être considéré comme un procédé de traitement cosmétique des cheveux en leur donnant une plus grande vigueur et un aspect meilleur.

Les exemples suivants sont destinés à illustrer l'invention sans pour autant présenter un caractère limitatif.

## EXEMPLE 1

On conditionne en kit deux compositions (A) et (B) renfermant respectivement :

| | |
|---|---|
| Composition (A) : Shampooing à l'anthraline conditionné en deux parties : | |
| 1A) Gel | |
| Anthraline<br>Silice colloïdale vendue par la Société DEGUSSA sous la dénomination "AEROSIL 200"<br>MIGLYOL 812 | 0,60 g<br>8,00 g<br>qsp 100,00 g |
| 2A) Partie aqueuse | |
| Lauryléther sulfate de sodium oxyéthyléné à l'aide de 2,2 moles d'oxyde d'éthylène<br>Acide lactique qs pH = 4<br>Eau | 12,00 g<br><br>qsp 100,00 g |

Au moment de l'emploi, on procède au mélange de 25% de la partie gel (1A) et de 75% de la partie aqueuse (2A).

On applique la composition (A) résultante sur le cuir chevelu. On laisse agir pendant environ 5 minutes à 1 heure, puis on émulsionne à l'aide d'eau et l'on rince abondamment à l'eau.

On applique ensuite la composition (B) :

| Composition (B) : Lotion au "Minoxidil" | |
|---|---|
| Minoxidil | 1,00 g |
| Propylèneglycol | 20,00 g |
| Alcool éthylique | 50,00 g |
| Eau | qsp 100,00 g |

EXEMPLE 2

On conditionne en kit deux compositions (A) et (B) renfermant respectivement :

| Composition (A) : | |
|---|---|
| Anthraline | 0,40 g |
| Acide salicylique | 0,40 g |
| Myristate d'isopropyle | qsp 100,00 g |

Le myristate d'isopropyle pouvant être remplacé par un mélange 50:50 d'octanoate de cétyle et d'octanoate de stéaryle.

On applique cette composition (A) sur le cuir chevelu. Après quelques minutes à une heure de pose, on effectue un shampooing conventionnel et on rince à l'eau.

On applique ensuite une composition (B) suivante :

| Composition (B) : | |
|---|---|
| Minoxidil | 2,00 g |
| Propylèneglycol | 5,00 g |
| Alcool éthylique | qsp 100,00 g |

EXEMPLE 3

On conditionne en kit deux compositions (A) et (B) renfermant respectivement :

| Composition (A) : | |
|---|---|
| Anthraline | 0,5 g |
| Myristate d'isopropyle | qsp 100,00 g |

| Composition (B) : | |
|---|---|
| Minoxidil | 1,33 g |
| Propylèneglycol | 20,00 g |
| Alcool éthylique | 50,00 g |
| Eau | qsp 100,00 g |

On réalise un mélange extemporané des deux compositions (A) et (B) à raison de 25% de la composition (A) pour 75% de la composition (B).

On applique le mélange résultant sur le cuir chevelu. On laisse agir quelques temps, puis on effectue un shampooing conventionnel.

EXEMPLE 4

On prépare la composition suivante que l'on applique sur le cuir chevelu.

| | |
|---|---|
| Anthraline | 0,03 g |
| Minoxidil | 1,00 g |
| Myristate d'isopropyle | 25,00 g |
| Propylèneglycol | 22,50 g |
| Alcool éthylique | qsp 100,00 g |

EXEMPLE 5

On conditionne en kit deux compositions (A) et (B) renfermant respectivement :

| Composition (A) : | |
|---|---|
| Acide (dihydroxy-1,8 anthrone-9)-yl 10 succinamique | 0,14 g |
| Propylèneglycol | qsp 100,00 g |

On applique cette composition (A) sur le cuir chevelu. Après quelques minutes de pose, on applique ensuite une composition (B) de composition :

| Composition (B) : | |
|---|---|
| Minoxidil | 1,00 g |
| Propylèneglycol | 20,00 g |
| Alcool éthylique | 50,00 g |
| Eau | qsp 100,00 g |

EXEMPLE 6

On conditionne en kit deux compositions (A) et (B) renfermant respectivement :

| Composition (A) : | |
|---|---|
| Dihydroxy-1,8 phényl-10 anthrone-9 | 9,00 g |
| Silice colloïdale vendue sous la dénomination AEROSIL 200 par la Société DEGUSSA | 7,00 g |
| Myristate d'isopropyle | qsp 100,00 g |

On applique ce gel sur le cuir chevelu. Après quelques minutes à une heure de pose, on effectue un shampooing conventionnel et on rince à l'eau.

On applique ensuite une composition (B) suivante :

| Composition (B) : | |
|---|---|
| Minoxidil | 2,00 g |
| Propylèneglycol | 5,00 g |
| Alcool éthylique | qsp 100,00 g |

EXEMPLE 7

On conditionne en kit deux compositions (A) et (B) renfermant respectivement :

| Composition (A) : | |
|---|---|
| Dipropionyloxy-1,8 propionyl-10-anthrone<br>Chlorure stanneux<br>Myristate d'isopropyle | 0,5 g<br>0,3 g<br>qsp 100,00 g |

On applique cette composition sur le cuir chevelu. Après quelques minutes à une heure de pose, on effectue un shampooing conventionnel et on rince à l'eau.

On applique ensuite une composition (B) suivante :

| Composition (B) : | |
|---|---|
| Minoxidil<br>Propylèneglycol<br>Alcool éthylique<br>Eau | 2,00 g<br>20,00 g<br>50,00 g<br>qsp 100,00 g |

EXEMPLE 8

On conditionne en kit deux compositions (A) et (B) renfermant respectivement :

| Composition (A) : | |
|---|---|
| Dipivaloyloxy-1,8 phényl-10 anthrone<br>Chlorure stanneux<br>Myristate d'isopropyle | 0,5 g<br>0,3 g<br>qsp 100,00 g |

On applique cette composition sur le cuir chevelu. Après quelques minutes à une heure de pose, on effectue un shampooing conventionnel et on rince à l'eau.

On applique ensuite une composition (B) suivante :

| Composition (B) : | |
|---|---|
| Minoxidil<br>Propylèneglycol<br>Alcool éthylique | 2,00 g<br>5,00 g<br>qsp 100,00 g |

**Revendications**

**1.** Association destinée à induire et à stimuler la croissance des cheveux et diminuer leur chute, caractérisée par le fait qu'elle comprend :
(a) un composant (A) contenant dans un milieu physiologiquement acceptable au moins un dérivé de 1,8-dihydroxy, 1,8-diacyloxy ou 1-hydroxy 8-acyloxy anthracène ou anthrone,
(b) un composant (B) contenant dans un milieu physiologiquement acceptable au moins un dérivé de pyrimidine,
répondant à la formule :

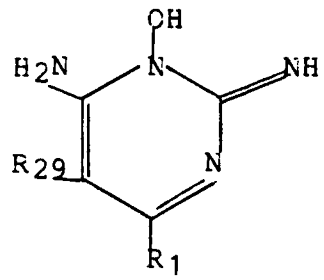

dans laquelle $R_1$ désigne un groupement

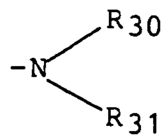

dans lequel $R_{30}$ et $R_{31}$ peuvent désigner indépendamment l'un de l'autre hydrogène, un groupement alkyle, alcényle, alkylaryle ou cycloalkyle, $R_{30}$ et $R_{31}$ peuvent également former conjointement un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholinyle et alkyl(inférieur)-4-pipérazidinyle, les groupements hétérocycliques pouvant être substitués sur les atomes de carbone par un à trois groupements alkyle inférieur, hydroxy ou alcoxy; le groupement $R_{29}$ désignant un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) étant présents dans une composition unique ou étant destinés à des applications séparées, soit simultanées, soit successives ou décalées dans le temps sur les cheveux et le cuir chevelu.

**2.** Association selon la revendication 1, caractérisée par le fait que le dérivé de 1,8-hydroxy et/ou acyloxy anthracène ou anthrone est choisi parmi :

- le 1,8,9-trihydroxy anthracène ou 1,8-dihydroxy 9-anthrone et son dimère,
- la 1,8,1′,8′-tétrahydroxy dianthrone,
- les 1,8-dihydroxy 9-anthrone substituées en position 10 par des groupements de formules :

i) 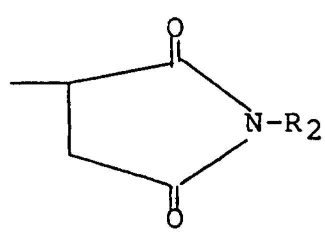

ii) 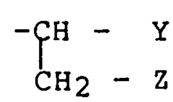

iii) 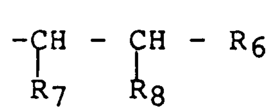

dans lesquelles $R_2$ représente un atome d'hydrogène, un radical alkyle, linéaire ou ramifié, ayant de 1 à 8 atomes de carbone, un radical mono- ou polyhydroxyalkyle linéaire ou ramifié, ayant 1 à 3 atomes de carbone, un radical carbamoyle ou un radical phényle; Y et Z désignent simultané-

ment un groupement $-CONR_3R_4$ ou bien désignent indépendamment l'un de l'autre, soit un groupement $-CONR_3R_4$ ou bien un groupement $CO_2R_5$, $R_3$, $R_4$ et $R_5$ représentant un atome d'hydrogène, un radical alkyle ayant de 1 à 8 atomes de carbone, un radical mono- ou polyhydroxyalkyle, éventuellement interrompu par un atome d'oxygène ayant 3 à 8 atomes de carbone, un radical cycloalkyle ayant de 3 à 6 atomes de carbone, ou bien $R_3$ et $R_4$ pris ensemble forment un radical divalent choisi parmi les radicaux suivants :

$-(CH_2)_n-$ où n est égal à 4 ou 5

$-(CH_2)_2-O-(CH_2)_2-$

ou $-(CH_2)_2-N(R_9)-(CH_2)_2-$

dans laquelle $R_9$ désigne un atome d'hydrogène, un radical méthyle ou hydroxy-2 éthyle; $R_6$ désigne un groupement $CO_2R_5$, CN, CHO, $CONH_2$ ou bien $CONH-CH_2OH$, dans lesquelles $R_5$ a la même signification que celle indiquée ci-dessus, $R_7$ et $R_8$ représentent indépendamment l'un de l'autre un atome d'hydrogène ou un radical méthyle ainsi que leurs sels et isomères optiques,

- les adducts de 1,8-dihydroxy 9-anthrone, comportant aux positions 9 et 10 les radicaux divalents suivants :

iv) 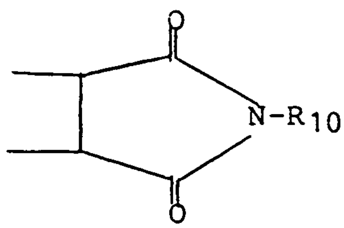

v) 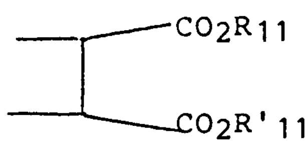

vi) 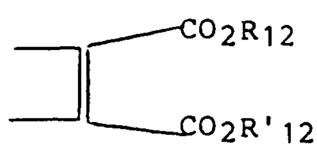

dans laquelle $R_{10}$, $R_{11}$ et $R'_{11}$ représentent un atome d'hydrogène, un radical alkyle inférieur, linéaire ou ramifié, ayant de 1 à 8 atomes de carbone, un radical monohydroxyalkyle éventuellement interrompu par un ou plusieurs atomes d'oxygène ayant 2 à 8 atomes de carbone, un radical cycloalkyle ayant 4 à 6 atomes de carbone, un radical phényle ou un radical benzyle, $R_{12}$ et $R'_{12}$, identiques ou différents, représentent un atome d'hydrogène, un radical alkyle inférieur, linéaire ou ramifié, ayant de 1 à 8 atomes de carbone à l'exclusion de $R_{12} = R'_{12} = CH_3$, un radical monohydroxyalkyle, éventuellement interrompu par un ou plusieurs atomes d'oxygène ayant 2 à 8 atomes de carbone, un radical phényle ou benzyle et les sels de ces composés;

- les 1,8-dihydroxy anthrones substituées en position 10 par les groupements :

vii) 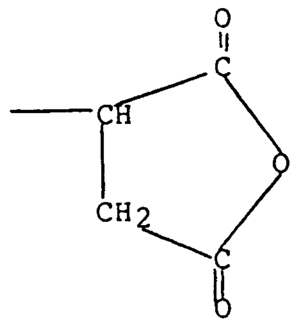

viii) 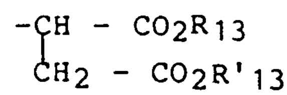

dans lesquels $R_{13}$ et $R'_{13}$, identiques ou différents, représentent un atome d'hydrogène ou un radical méthyle ou éthyle, ou leurs isomères; ou par le groupement de formule :

ix) 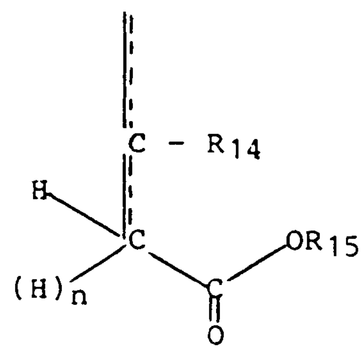

dans laquelle, $R_{14}$ représente un atome d'hydrogène ou le radical $COR'_{15}$, $R_{15}$ et $R'_{15}$, identiques ou différents, représentant un atome d'hydrogène, un radical alkyle, linéaire ou ramifié, ayant de 1 à 6 atomes de carbone, un radical cycloalkyle ayant de 4 à 6 atomes de carbone ou un radical benzyle, ou bien $R_{14}$ et $R_{15}$, pris ensemble, forment un radical

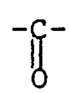

et n est égal à 0 ou 1, ainsi que leur isomères;

- les aryl-10 dihydroxy-1,8 anthrones dont le groupement aryl répond à la formule suivante :

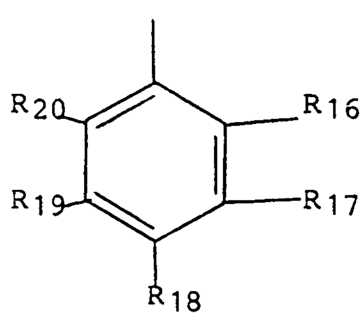

dans laquelle $R_{16}$, $R_{17}$, $R_{18}$, $R_{19}$, $R_{20}$, identiques ou différents, représentent un atome d'hydrogène, un atome d'halogène, le radical $CF_3$, une fonction hydroxyle, un radical alkyle ou cycloalkyle inférieur, un radical hydroxyalkyle inférieur, un radical alcoxy inférieur, une fonction nitrile, un radical

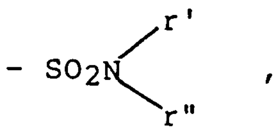

,

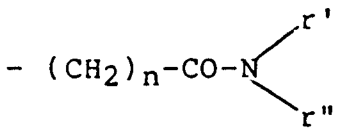

,

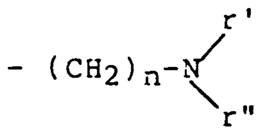

, $-(CH_2)_n-CO_2R'$, ou

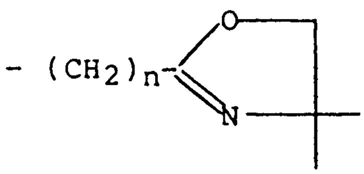

,

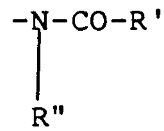

r′, r″, identiques ou différents, représentent un atome d'hydrogène ou un radical alkyle inférieur, n est 0 ou un nombre entier de 1 à 3 inclus, et R′ et R″ représentent un atome d'hydrogène, un radical alkyle inférieur, linéaire ou ramifié, ou un radical aryle éventuellement substitué(s);

- les diacyloxy-1,8 acyl-10 anthrones de formule générale :

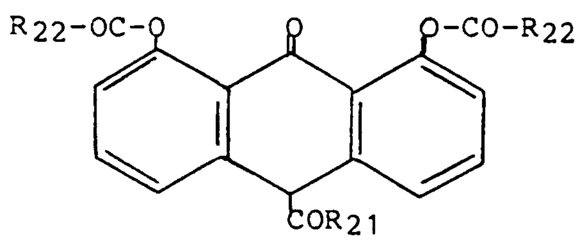

dans laquelle :

$R_{21}$, $R_{22}$, identiques ou différents, représentent un radical alkyle, linéaire ou ramifié, ayant de 1 à 15 atomes de carbone, un radical cycloalkyle de 3 à 6 atomes de carbone, un radical furyl-2 ou -3, un radical pyridyl-3 ou -4, un radical thiényl-2 ou un radical aromatique de formule :

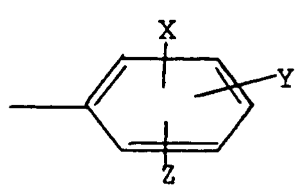

dans laquelle X, Y, Z, identiques ou différents, représentent un atome d'hydrogène, un radical alkyle inférieur, un radical trifluorométhyle, un radical alcoxy inférieur, un atome d'halogène, un groupement nitro ou hydroxyle;

- l'acétyl-10 triacétoxy-1,8,9 anthracène de formule :

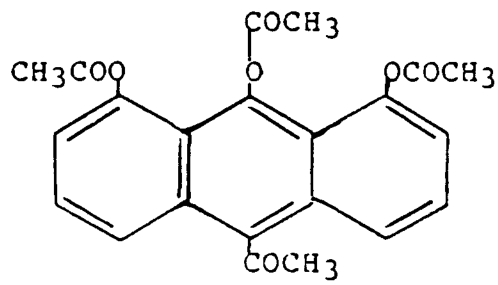

- les hydroxy-1, acyloxy-8 acyl-10 anthrones de formule générale :

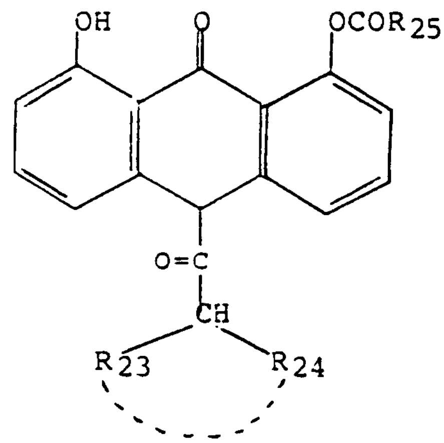

dans laquelle :
$R_{23}$ représente un atome d'hydrogène ou un radical alkyle inférieur linéaire;
$R_{24}$ représente un atome d'hydrogène, un radical alkyle linéaire ou ramifié, ayant de 1 à 17 atomes de carbone, un radical alkényle, linéaire ou ramifié, ayant de 2 à 17 atomes de carbone ou $R_{23}$ ou $R_{24}$ pris ensemble forment avec l'atome de carbone auquel ils sont attachés, un radical cycloalkyle ayant de 3 à 6 atomes de carbone;
$R_{25}$ représente un radical alkyle, linéaire ou ramifié, ayant de 1 à 18 atomes de carbone, un radical alkényle, linéaire ou ramifié, ayant de 2 à 18 atomes de carbone, un radical cycloalkyle ayant de 3 à 6 atomes de carbone, un radical furyle-2 ou -3, un radical pyridyle-3 ou -4, un radical thiényl-2 ou un radical aromatique de formule :

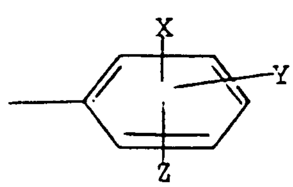

dans laquelle X, Y, Z, indentiques ou différents, représentent un atome d'hydrogène, un radical alkyle inférieur, un radical trifluorométhyle, un radical alcoxy inférieur, un atome d'halogène, un groupement nitro ou un groupe hydroxyle;
- le triacétoxy-1,8,9 anthracène de formule :

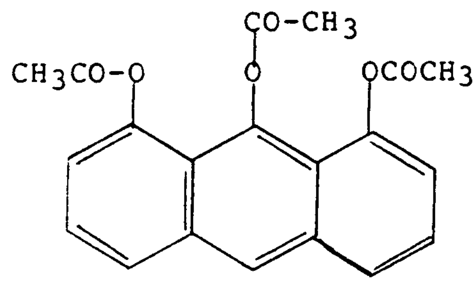

- les mono-, di- et tri-esters de dihydroxy-1,8 phényl-10 anthrone-9 ou anthranol-9 de formule générale suivante :

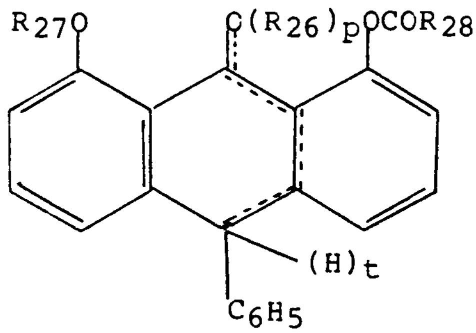

(I)

dans laquelle :

p est 0 ou 1;

. lorsque p = 0, t = 1 et $R_{27}$ représente un atome d'hydrogène ou $-COR_3$;

. lorsque p = 1, t = 0 et $R_{26}$ et $R_{27}$ représentent, indépendamment l'un de l'autre, un atome d'hydrogène ou $-COR_{28}$;

$R_{28}$ représentant un radical alkyle, linéaire ou ramifié, ayant de 1 à 17 atomes de carbone, un radical cycloalkyle ou un radical phényle éventuellement substitué par un alkyle inférieur, un alcoxy inférieur, un alcoxy inférieur, un atome d'halogène, une fonction nitro, un radical $-CF_3$ ou par une fonction hydroxyle,

et les mélanges desdits esters.

**3.** Association selon la revendication 1, caractérisée par le fait que le dérivé de pyrimidine de formule (I) est choisi parmi les composés dans lesquels $R_{29}$ désigne hydrogène et $R_1$ représente un groupement

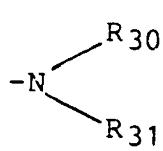

,

dans lequel $R_{30}$ et $R_{31}$ forment un cycle pipéridinyle.

**4.** Association selon l'une quelconque des revendications 1 à 3, caractérisée par le fait que le composé de formule (I) est constitué par l'amino-6 dihydro-1,2 hydroxy-1 imino-2 pipéridino-4 pyrimidine ou "Minoxidil".

**5.** Association selon l'une quelconque des revendications 1 à 3, caractérisée par le fait que le dérivé de 1,8-hydroxy et/ou acyloxy anthracène ou anthrone est constitué par le 1,8,9-trihydroxy anthracène ou 1,8-dihydroxy 9-anthrone ou "anthraline".

**6.** Association selon l'une quelconque des revendications 1 à 4, caractérisée par le fait que le composant (A) contient le dérivé de 1,8-hydroxy et/ou acyloxy anthracène ou anthrone dans des proportions

comprises entre 0,01 et 10% en poids par rapport au poids du composant (A), et de préférence comprises entre 0,1 et 1% et plus particulièrement entre 0,1 et 0,5% en poids.

**7.** Association selon l'une quelconque des revendications 1 à 5, caractérisée par le fait que le composant (B) contient le composé de formule (I) dans des proportions comprises entre 0,05 et 10% en poids par rapport au poids total de la composition et de préférence entre 0,05 et 5%, et en particulier entre 0,5 et 4%.

**8.** Association selon l'une quelconque des revendications 1 à 6, caractérisée par le fait que le rapport pondéral entre le dérivé de pyrimidine de formule (I) et le 1,8-hydroxy et/ou acyloxy anthracène ou anthrone est compris entre 2/1 et 10/1.

**9.** Association selon l'une quelconque des revendications 1 à 8, caractérisée par le fait qu'elle se présente sous forme d'une composition unique contenant les composants (A) et (B), dans laquelle le dérivé de 1,8-hydroxy et/ou acyloxy anthracène ou anthrone est présent dans des proportions comprises de préférence entre 0,01 et 5% en poids par rapport au poids total de la composition et que le dérivé de pyrimidine de formule (I) est présent dans des proportions comprises entre 0,05 et 6% en poids par rapport au poids total de la composition.

**10.** Association selon l'une quelconque des revendications 1 à 9, caractérisée par le fait que les milieux physiologiquement acceptables sont constitués par un ou plusieurs solvants anhydres ou un mélange d'eau et d'un ou plusieurs solvant(s), les solvants étant choisis parmi les alcools inférieurs, les alkylèneglycols ou les alkyléthers d'alkylèneglycols ou de dialkylèneglycols.

**11.** Association selon l'une quelconque des revendications 1 à 10, caractérisée par le fait que l'un au moins des milieux physiologiquement acceptables est épaissi au moyen d'agents épaississants et/ou gélifiants.

**12.** Association selon l'une quelconque des revendications 1 à 11, caractérisée par le fait que l'un au moins des composants (A) ou (B) contient également un adjuvant cosmétiquement ou pharmaceutiquement acceptable choisi parmi des agents conservateurs, des agents complexants, des colorants, des agents alcalinisants ou acidifiants, des agents tensio-actifs, anioniques, cationiques, non ioniques ou amphotères ainsi que leurs mélanges, des polymères anioniques, cationiques, non ioniques ou amphotères ainsi que leurs mélanges.

**13.** Procédé de traitement cosmétique des cheveux ou du cuir chevelu, caractérisé par le fait que l'on applique les composants (A) et (B) de l'association telle que définie dans l'une quelconque des revendications 1 à 12, de façon séparée, soit simultanément, soit de façon successive ou décalée dans le temps.

**14.** Dispositif à plusieurs compartiments ou "kit", caractérisé par le fait qu'il comprend dans un premier compartiment un composant (A) contenant dans un milieu physiologiquement acceptable, un dérivé de 1,8-hydroxy et/ou acyloxy anthracène ou anthrone tel que défini dans la revendication 1 ou 2 et un second compartiment contenant un composant (B) contenant dans un milieu physiologiquement acceptable un dérivé de pyrimidine répondant à la formule (I).

**15.** Association selon l'une quelconque des revendications 1 à 12 pour son application thérapeutique dans le traitement de la chute des cheveux.

**16.** Utilisation de l'association selon l'une quelconque des revendications 1 à 12 pour la préparation d'un médicament destiné à être utilisé dans le traitement thérapeutique de la chute des cheveux.

### Claims

**1.** Combination intended to induce and to stimulate hair growth and to reduce its loss, characterized in that it comprises:

(a) a component (A) containing at least one derivative of 1,8-dihydroxy-, 1,8-diacyloxy- or 1-hydroxy-8-acyloxyanthracene or anthrone, in a physiologically acceptable medium,

(b) a component (B) containing, in a physiologically acceptable medium, at least one pyrimidine derivative corresponding to the formula:

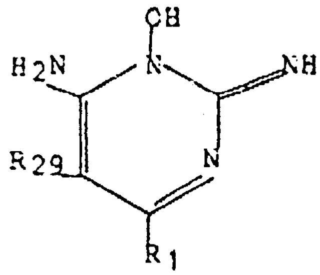

(I)

in which $R_1$ denotes a group

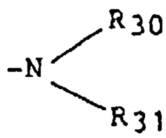

in which $R_{30}$ and $R_{31}$ may, independently of each other, denote hydrogen or an alkyl, alkenyl, alkylaryl or cycloalkyl group; $R_{30}$ and $R_{31}$ can also conjointly, with the nitrogen atom to which they are bonded, form a heterocyclic ring chosen from aziridinyl, azetidinyl, pyrrolidinyl, piperidyl, hexahydroazeopinyl, heptamethyleneimine, octamethyleneimine, morpholinyl and 4-(lower)-alkylpiperazidinyl groups, it being possible for the heterocyclic groups to be substituted on the carbon atoms by 1 to 3 lower alkyl, hydroxyl or alkoxy groups; the group $R_{29}$ denoting a hydrogen atom or an alkyl, alkenyl, alkylalkoxy, cycloalkyl, aryl, alkylaryl, arylalkyl, alkylarylalkyl, alkoxyarylalkyl or haloarylalkyl group, as well as the addition salts of physiologically acceptable acids, the components (A) and (B) being present in a sole composition or being intended for separate applications, either simultaneous or successive or spaced in time, to hair and to the scalp.

2. Combination according to Claim 1, characterized in that the derivative of 1,8-hydroxy and/or acyloxy anthracene or anthrone is chosen from:
- 1,8,9-trihydroxyanthracene or 1,8-dihydroxy-9-anthrone and its dimer,
- 1,8,1',8'-tetrahydroxydianthrone,
- 1,8-dihydroxy-9-anthrones substituted in position 10 by groups of formulae:

(i)

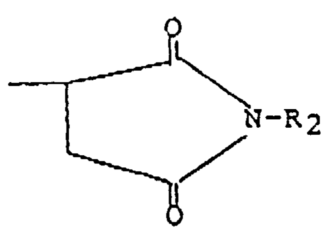

(ii)

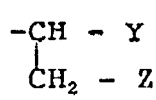

(iii)

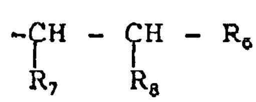

in which $R_2$ denotes a hydrogen atom, a linear or branched alkyl radical containing from 1 to 8 carbon atoms, a linear or branched mono- or polyhydroxyalkyl radical containing from 1 to 3

carbon atoms, a carbamoyl radical or a phenyl radical,

Y and Z denote simultaneously a $-CONR_3R_4$ group or else denote, independently of each other, either a $-CONR_3R_4$ group or else a $CO_2R_5$ group, with $R_3$, $R_4$ and $R_5$ denoting a hydrogen atom, an alkyl radical containing from 1 to 8 carbon atoms, a mono- or polyhydroxyalkyl radical, optionally interrupted by an oxygen atom, containing from 3 to 8 carbon atoms, a cycloalkyl radical containing from 3 to 6 carbon atoms, or else $R_3$ and $R_4$ taken together form a divalent radical chosen from the following radicals:

$-(CH_2)_n-$ where n is equal to 4 or 5

$-(CH_2)_2-O-(CH_2)_2-$

or 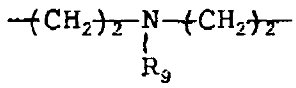

in which $R_9$ denotes a hydrogen atom or a methyl or 2-hydroxyethyl radical;

$R_6$ denotes a $CO_2R_5$, CN, CHO, $CONH_2$ or $CONH-CH_2OH$ group, in which $R_5$ has the same meaning as that indicated above, and $R_7$ and $R_8$ denote, independently of each other, a hydrogen atom or a methyl radical, as well as their salts and optical isomers,

- 1,8-dihydroxy-9-anthrone adducts comprising the following divalent radicals in positions 9 and 10:

iv) 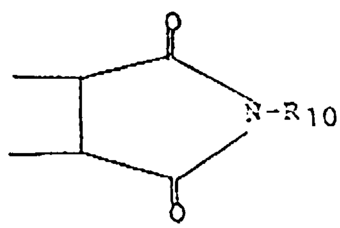

v) 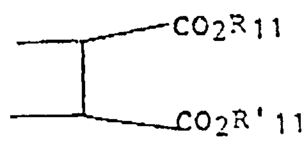

vi) 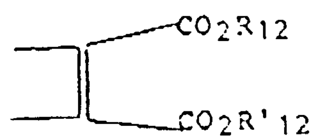

in which $R_{10}$, $R_{11}$ and $R'_{11}$ denote a hydrogen atom, a linear or branched lower alkyl radical containing from 1 to 8 carbon atoms, a monohydroxyalkyl radical optionally interrupted by one or more oxygen atoms, containing from 2 to 8 carbon atoms, a cycloalkyl radical containing from 4 to 6 carbon atoms, a phenyl radical or a benzyl radical, $R_{12}$ and $R'_{12}$, which are identical or different, denote a hydrogen atom, a linear or branched lower alkyl radical containing from 1 to 8 carbon atoms except for $R_{12} = R'_{12} = CH_3$, a monohydroxyalkyl radical, optionally interrupted by one or more oxygen atoms, containing from 2 to 8 carbon atoms, a phenyl or benzyl radical and the salts of these compounds:

- 1,8-dihydroxy-9-anthrones substituted in position 10 by the groups:

vii) 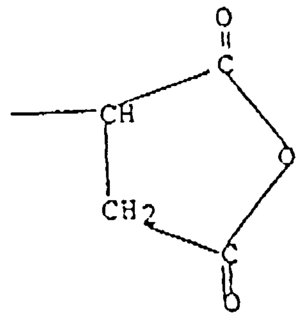

viii) 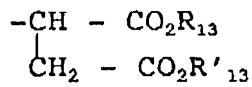

in which $R_{13}$ and $R'_{13}$, which are identical or different, denote a hydrogen atom or a methyl or ethyl radical, or their isomers,

or by the group of formula:

ix) 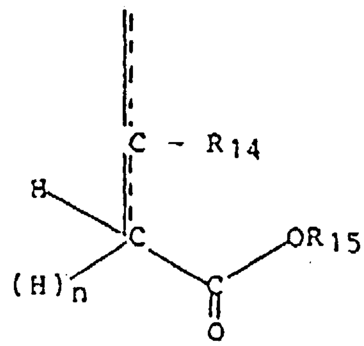

in which $R_{14}$ denotes a hydrogen atom or the radical $COR'_{15}$, with $R_{15}$ and $R'_{15}$, which are identical or different, denoting a hydrogen atom, a linear or branched alkyl radical containing from 1 to 6 carbon atoms, a cycloalkyl radical containing from 4 to 6 carbon atoms or a benzyl radical, or else $R_{14}$ and $R_{15}$, taken together, form a radical

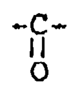

and n is equal to 0 or 1, as well as their isomers.

- the 10-aryl-1,8-dihydroxy anthrones in which the aryl group corresponds to the following formula:

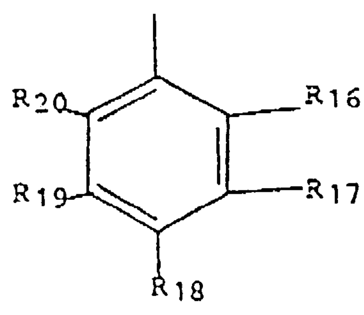

in which $R_{16}$, $R_{17}$, $R_{18}$, $R_{19}$ and $R_{20}$ which are identical or different, denote a hydrogen atom, a

halogen atom, the $CF_3$ radical, a hydroxyl group, a lower alkyl or cycloalkyl radical, a lower hydroxyalkyl radical, a lower alkoxy radical, a nitrile group, a radical

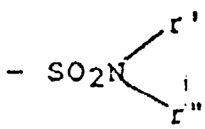 ,

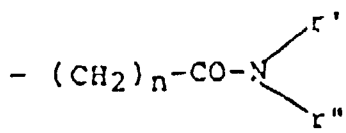 , 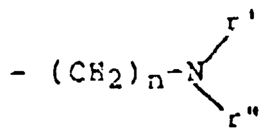 , $-(CH_2)_n-CO_2R'$, or

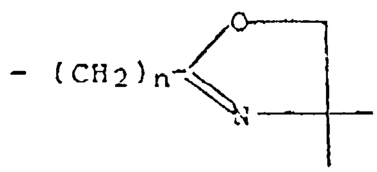 , 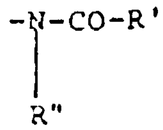

r' and r'', which are identical or different, denote a hydrogen atom or a lower alkyl radical, n is 0 or an integer from 1 to 3 inclusive, and R' and R'' denote a hydrogen atom, a linear or branched lower alkyl radical or an aryl radical, which is or are optionally substituted;

- 1,8-diacyloxy-10-acylanthrones of general formula:

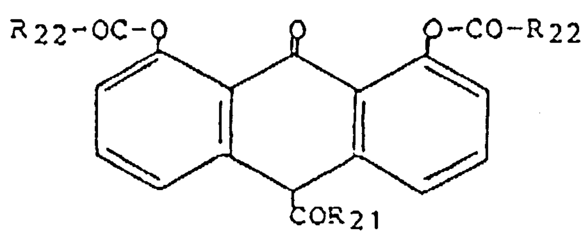

in which:
$R_{21}$ and $R_{22}$, which are identical or different, denote a linear or branched alkyl radical containing from 1 to 15 carbon atoms, a cycloalkyl radical with 3 to 6 carbon atoms, a 2- or 3-furyl radical, a 3- or 4-pyridyl radical, a 2-thienyl radical or an aromatic radical of formula:

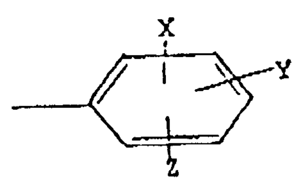

in which X, Y and Z, which are identical or different, denote a hydrogen atom, a lower alkyl radical, a trifluoromethyl radical, a lower alkoxy radical, a halogen atom or a nitro or hydroxyl group;

- 10-acetyl-1,8,9-triacetoxyanthracene of formula:

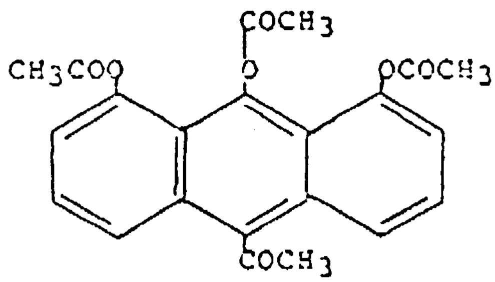

- 1-hydroxy-8-acyloxy-10-acyl anthrones of general formula:

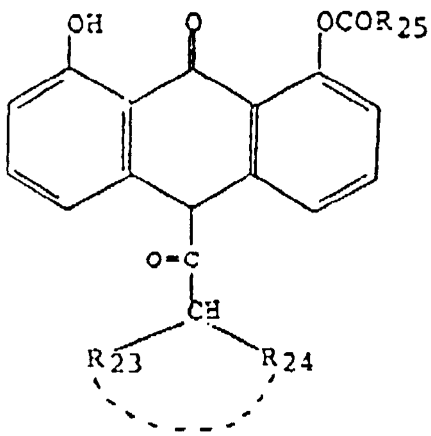

in which:
$R_{23}$ denotes a hydrogen atom or a linear lower alkyl radical;
$R_{24}$ denotes a hydrogen atom, a linear or branched alkyl radical containing from 1 to 17 carbon atoms, a linear or branched alkenyl radical containing from 2 to 17 carbon atoms, or $R_{23}$ and $R_{24}$ taken together form, together with the carbon atom to which they are attached, a cycloalkyl radical containing from 3 to 6 carbon atoms;
$R_{25}$ denotes a linear or branched alkyl radical containing from 1 to 18 carbon atoms, a linear or branched alkenyl radical containing from 2 to 18 carbon atoms, a cycloalkyl radical containing from 3 to 6 carbon atoms, a 2- or 3-furyl radical, a 3- or 4-pyridyl radical, a 2-thienyl radical or an aromatic radical of formula:

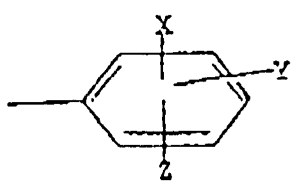

in which X, Y and Z, which are identical or different, denote a hydrogen atom, a lower alkyl radical, a trifluoromethyl radical, a lower alkoxy radical, a halogen atom, a nitro group or a hydroxyl group;
- 1,8,9-triacetoxyanthracene of formula:

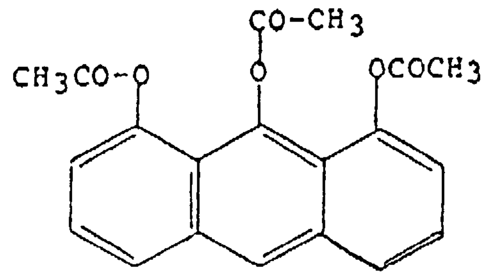

- mono-, di- and triesters of 1,8-dihydroxy-10-phenyl-9-anthrone or 9-anthranol of the following general formula:

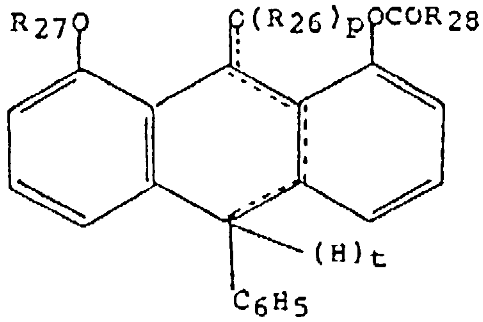

(I)

in which:

p is 0 or 1:

when p = 0, t = 1 and $R_{27}$ denotes a hydrogen atom or $-COR_3$;

when p = 1, t = 0 and $R_{26}$ and $R_{27}$ denote, independently of each other, a hydrogen atom or $-COR_{28}$;

$R_{28}$ denoting a linear or branched alkyl radical containing from 1 to 17 carbon atoms, a cycloalkyl radical, or a phenyl radical optionally substituted by a lower alkyl, a lower alkoxy, a halogen atom, a nitro group, a $-CF_3$ radical or by a hydroxyl group,

and mixtures of the said esters.

**3.** Combination according to Claim 1, characterized in that the pyrimidine derivative of formula (I) is chosen from the compounds in which $R_{29}$ denotes hydrogen and $R_1$ denotes a group

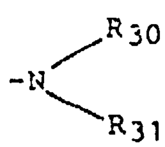

,

in which $R_{30}$ and $R_{31}$ form a piperidyl ring.

**4.** Combination according to any one of Claims 1 to 3, characterized in that the compound of formula (I) consists of 6-amino-1,2-dihydro-1-hydroxy-2-imino-4-piperidinopyrimidine or "Minoxidil".

**5.** Combination according to any one of Claims 1 to 3, characterized in that the derivative of 1,8-hydroxy and/or acyloxy anthracene or anthrone consists of 1,8,9-trihydroxyanthracene or 1,8-dihydroxy-9-anthrone or "anthraline".

**6.** Combination according to any one of Claims 1 to 4, characterized in that the component (A) contains the derivative of 1,8-hydroxy and/or acyloxy anthracene or anthrone in proportions of between 0.01 and

10% by weight relative to the weight of component (A), and preferably between 0.1 and 1% and more particularly between 0.1 and 0.5% by weight.

**7.** Combination according to any one of Claims 1 to 5, characterized in that the component (B) contains the compound of formula (I) in proportions of between 0.05 and 10% by weight relative to the total weight of the composition and preferably between 0.05 and 5% and in particular between 0.5 and 4%.

**8.** Combination according to any one of Claims 1 to 6, characterized in that the weight ratio of the pyrimidine derivative of formula (I) to the 1,8-hydroxy and/or acyloxy anthracene or anthrone is between 2/1 and 10/1.

**9.** Combination according to any one of Claims 1 to 8, characterized in that it is present in the form of a single composition containing the components (A) and (B) in which the derivative of 1,8-hydroxy and/or acyloxy anthracene or anthrone is present in proportions of preferably between 0.01 and 5% by weight relative to the total weight of the composition and that the pyrimidine derivative of formula (I) is present in proportions of between 0.05 and 6% by weight relative to the total weight of the composition.

**10.** Combination according to any one of Claims 1 to 9, characterized in that the physiologically acceptable media consist of one or more anhydrous solvents or a mixture of water and of one or more solvent(s), the solvents being chosen from lower alcohols, alkylene glycols or alkylene glycol or dialkylene glycol alkyl ethers.

**11.** Combination according to any one of Claims 1 to 10, characterized in that at least one of the physiologically acceptable media is thickened by means of thickening and/or gelling agents.

**12.** Combination according to any one of Claims 1 to 11, characterized in that at least one of the components (A) and (B) also contains a cosmetically or pharmaceutically acceptable adjuvant chosen from preserving agents, complexing agents, colorants, alkalifying or acidifying agents, anionic, cationic, nonionic or amphoteric surface-active agents and mixtures thereof, and anionic, cationic, nonionic or amphoteric polymers and mixtures thereof.

**13.** Process for cosmetic treatment of hair or of the scalp, characterized in that the components (A) and (B) of the combination such as defined in any one of Claims 1 to 12 are applied separately, either simultaneously or successively or spaced in time.

**14.** Device with a number of compartments or a kit, characterized in that it comprises, in a first compartment, a component (A) containing a derivative of 1,8-hydroxy and/or acyloxy anthracene or anthrone such as defined in Claim 1 or 2, in a physiologically acceptable medium, and a second compartment containing a component (B) containing a pyrimidine derivative corresponding to formula (I), in a physiologically acceptable medium.

**15.** Combination according to any one of Claims 1 to 12, for its therapeutic application in the treatment of hair loss.

**16.** Use of the combination according to any one of Claims 1 to 12 for the preparation of a medication intended to be employed in the therapeutic treatment of hair loss.

**Patentansprüche**

**1.** Assoziat zum Induzieren und Stimulieren des Haarwachstums und zum Vermindern des Haarausfalls, dadurch **gekennzeichnet,** daß
es umfaßt:
(a) einen Bestandteil (A), enthaltend in einem physiologisch verträglichen Milieu mindestens ein Derivat von 1,8-Dihydroxy-, 1,8-Diacyloxy- oder 1-Hydroxy-8-acyloxyanthracen oder -anthron,
(b) einen Bestandteil (B), enthaltend in einem physiologisch verträglichen Milieu mindestens ein Pyrimidinderivat der Formel (I) sowie die physiologisch verträglichen Säureadditionssalze davon:

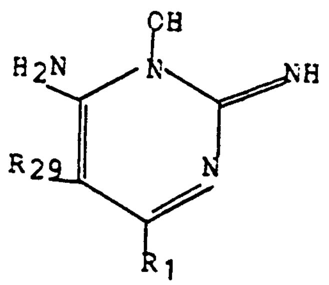

(I)

worin $R_1$ eine

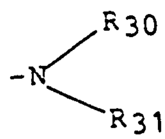

-Gruppe darstellt,
worin $R_{30}$ und $R_{31}$, unabhängig voneinander, Wasserstoff, eine Alkyl-, Alkenyl-, Alkylaryl- oder Cycloalkylgruppe darstellen, wobei $R_{30}$ und $R_{31}$ auch zusammen mit dem Stickstoffatom, an das sie gebunden sind, einen Heterozyklus bilden können, ausgewählt aus Aziridinyl- Azetidinyl-, Pyrrolidinyl-, Piperidinyl-, Hexahydroazepinyl-, Heptamethylenimin-, Octamethylenimin-, Morpholinyl- und Niedrigalkyl-4-piperazidinylgruppen, wobei die heterozyklischen Gruppen an den Kohlenstoffatomen durch eine bis drei Niedrigalkyl-, Hydroxy- oder Alkoxygruppen substituiert sein können; und worin die $R_{29}$-Gruppe ein Wasserstoffatom, eine Alkyl-, Alkenyl-, Alkylalkoxy-, Cycloalkyl-, Aryl-, Alkylaryl-, Arylalkyl-, Alkylarylalkyl-, Alkoxyarylalkyl- oder Haloarylalkylgruppe darstellt,
wobei die Bestandteile (A) und (B) in einer einzigen Zusammensetzung vorliegen oder zu getrennten Aufbringungen auf die Haare oder die behaarte Haut bestimmt sind, sei es gleichzeitig, sei es aufeinanderfolgend oder nach einem Zeitablauf.

**2.** Assoziat gemäß Anspruch 1,
dadurch **gekennzeichnet,** daß
das Derivat von 1,8-Hydroxy- und/oder -acyloxyanthracen oder -anthron ausgewählt ist unter:
- 1,8,9-Trihydroxyanthracen oder 1,8-Dihydroxy-9-anthron und seinem Dimeren,
- 1,8,1',8'-Tetrahydroxydianthron,
- 1,8-Dihydroxy-9-anthronen, substituiert in Position 10 durch Gruppen der Formeln:

i) 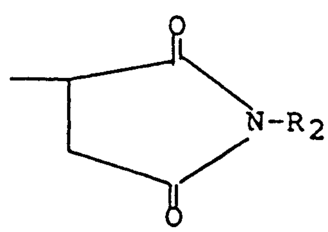

ii) 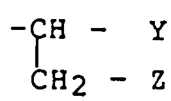

iii) 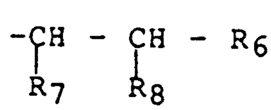

in denen $R_2$ ein Wasserstoffatom, einen linearen oder verzweigten Alkylrest mit 1 bis 8 Kohlenstoffatomen, einen linearen oder verzweigten Mono- oder Polyhydroxyalkylrest mit 1 bis 3 Kohlenstoffatomen, einen Carbamoyl- oder Phenylrest darstellt, Y und Z gleichzeitig eine $-CONR_3R_4$-Gruppe bedeuten oder auch, unabhängig voneinander, entweder eine $-CONR_3R_4$-Gruppe oder auch eine $-CO_2R_5$- Gruppe darstellen, wobei $R_3$, $R_4$ und $R_5$ ein Wasserstoffatom, einen Alkylrest mit 1 bis 8 Kohlenstoffatomen, einen gegebenenfalls durch ein Sauerstoffatom unterbrochenen Mono- oder Polyhydroxyalkylrest mit 3 bis 8 Kohlenstoffatomen, einen Cycloalkylrest mit 3 bis 6 Kohlenstoffatomen darstellen, oder wobei $R_3$ und $R_4$, zusammengenommen, auch einen divalenten Rest bilden, ausgewählt unter den folgenden Resten:

$-(CH_2)_n-$ worin n gleich 4 oder 5 ist,

$-(CH_2)_2-O-(CH_2)_2-$ oder

$-(CH_2)_2-N-(CH_2)_2-$

$\quad\quad\quad\quad | $

$\quad\quad\quad\quad R_9$

worin $R_9$ ein Wasserstoffatom, einen Methyl- oder Hydroxy-2-ethylrest bedeutet; und in denen $R_6$ eine $-CO_2R_5$-, -CN-, -CHO-, $-CONH_2$- oder auch $-CONH-CH_2OH$-Gruppe darstellt, worin $R_5$ dieselbe Bedeutung wie oben angegeben hat, $R_7$ und $R_8$, unabhängig voneinander, ein Wasserstoffatom oder einen Methylrest darstellen,
sowie deren Salze und optischen Isomere,

- den Addukten von 1,8-Dihydroxy-9-anthron, die in den Positionen 9 und 10 die folgenden divalenten Reste aufweisen:

iv) 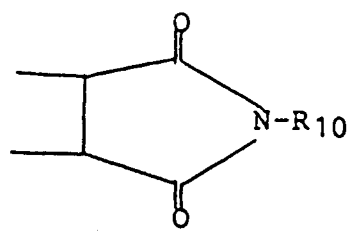

v) 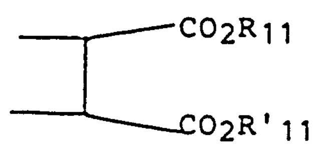

vi) 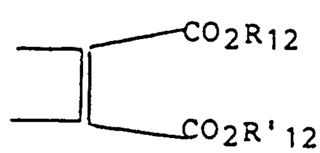

worin $R_{10}$, $R_{11}$ und $R'_{11}$ ein Wasserstoffatom, einen linearen oder verzweigten Niedrigalkylrest mit 1 bis 8 Kohlenstoffatomen, einen gegebenenfalls durch ein oder mehrere Sauerstoffatom(e) unterbrochenen Monohydroxyalkylrest mit 2 bis 8 Kohlenstoffatomen, einen Cycloalkylrest mit 4 bis 6 Kohlenstoffatomen, einen Phenyl- oder Benzylrest, $R_{12}$ und $R'_{12}$, gleich oder verschieden, ein Wasserstoffatom, einen linearen oder verzweigten Niedrigalkylrest mit 1 bis 8 Kohlenstoffatomen, ausgenommen $R_{12} = R'_{12} = CH_3$, einen gegebenenfalls durch ein oder mehrere Sauerstoffatom(e) unterbrochenen Monohydroxyalkylrest mit 2 bis 8 Kohlenstoffatomen, einen Phenyl- oder Benzylrest bedeuten,
und die Salze dieser Verbindungen,

- 1,8-Dihydroxyanthronen, substituiert in Position 10 durch die Gruppen:

vii) 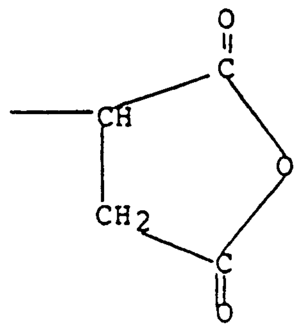

viii) 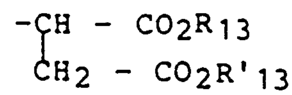

worin $R_{13}$ und $R'_{13}$, gleich oder verschieden, ein Wasserstoffatom oder einen Methyl- oder Ethylrest bedeuten, oder deren Isomere; oder substituiert durch die Gruppe der Formel:

ix) 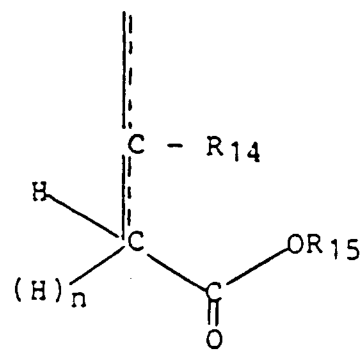

worin $R_{14}$ ein Wasserstoffatom oder den $-COR'_{15}$-Rest bedeutet, wobei $R_{15}$ und $R'_{15}$, gleich oder verschieden, ein Wasserstoffatom, einen linearen oder verzweigten Alkylrest mit 1 bis 6 Kohlenstoffatomen, einen Cycloalkylrest mit 4 bis 6 Kohlenstoffatomen oder einen Benzylrest darstellen oder $R_{14}$ und $R_{15}$, zusammengenommen, auch einen Rest

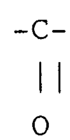

bilden, und worin n 0 oder 1 ist,
sowie deren Isomere;

- Aryl-10-dihydroxy-1,8-anthronen, worin die Arylgruppe der folgenden Formel entspricht:

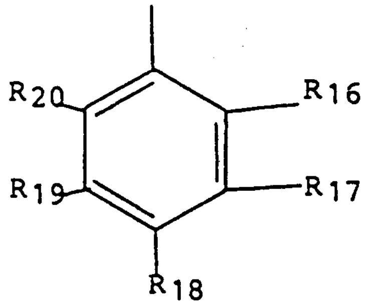

worin $R_{16}$, $R_{17}$, $R_{18}$, $R_{19}$, $R_{20}$, gleich oder verschieden ein Wasserstoffatom, ein Halogenatom, $CF_3$, eine Hydroxylfunktion, einen Niedrigalkyl- oder -cycloalkylrest, einen Hydroxyniedrigalkylrest, einen Niedrigalkoxyrest, eine Nitrilfunktion, einen Rest

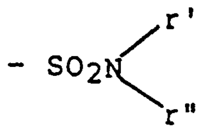 ,

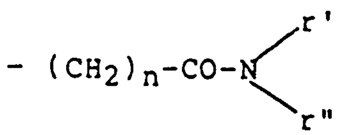 , 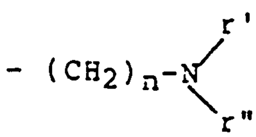 ,

$-(CH_2)_n-CO_2R'$, oder

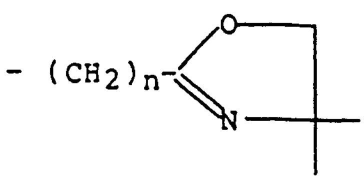 , 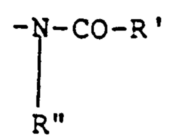

darstellen,
wori r' und r'', gleich oder verschieden, ein Wasserstoffatom oder einen Niedrigalkylrest darstellen, n 0 oder eine ganze Zahl von 1 bis 3 ist und R' und R'' ein Wasserstoffatom, einen linearen oder verzweigten Niedrigalkylrest oder einen gegebenenfalls ein- oder mehrfach substituierten Arylrest darstellen;

- Diacyloxy-1,8-acyl-10-anthronen der allgemeinen Formel:

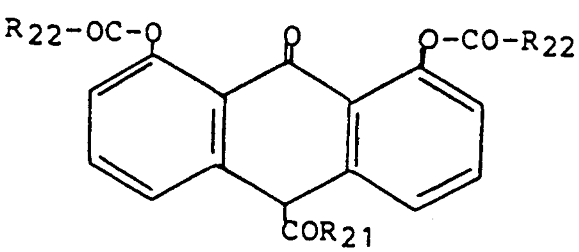

worin $R_{21}$, $R_{22}$, gleich oder verschieden, einen linearen oder verzweigten Alkylrest mit 1 bis 15

Kohlenstoffatomen, einen Cycloalkylrest mit 3 bis 6 Kohlenstoffatomen, einen Furyl-2- oder -3-Rest, einen Pyridyl-3 oder -4-Rest, einen Thienyl-2-Rest oder einen aromatischen Rest der Formel darstellen:

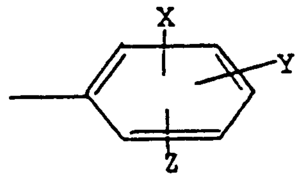

worin X, Y, Z, gleich oder verschieden, ein Wasserstoffatom, einen Niedrigalkylrest, einen Trifluormethylrest, einen Niedrigalkoxyrest, einen Halogenatom, eine Nitro- oder Hydroxylgruppe darstellen;

- Acetyl-10-triacetoxy-1,8,9-anthracen der Formel:

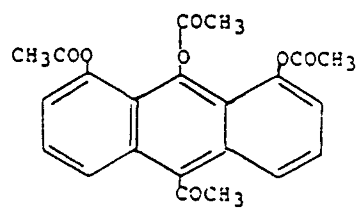

- Hydroxy-1-acyloxy-8-acyl-10-anthronen der allgemeinen Formel:

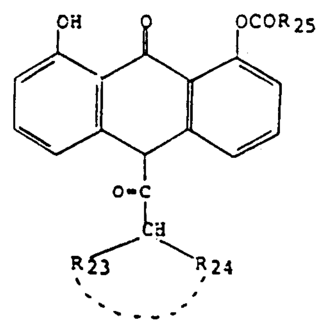

worin:
$R_{23}$ ein Wasserstoffatom oder einen linearen Niedrigalkylrest darstellt;
$R_{24}$ ein Wasserstoffatom, einen linearen oder verzweigten Alkylrest mit 1 bis 17 Kohlenstoffatomen, einen linearen oder verzweigten Alkenylrest mit 2 bis 17 Kohlenstoffatomen darstellt oder $R_{23}$ oder $R_{24}$, zusammen mit dem Kohlenstoffatom, an das sie gebunden sind, einen Cycloalkylrest mit 3 bis 6 Kohlenstoffatomen bilden,
$R_{25}$ einen linearen oder verzweigten Alkylrest mit 1 bis 18 Kohlenstoffatomen, einen linearen oder verzweigten Alkenylrest mit 2 bis 18 Kohlenstoffatomen, einen Cycloalkylrest mit 3 bis 6 Kohlenstoffatomen, einen Furyl-2- oder -3-Rest, einen Pyridyl-3- oder -4-Rest, einen Thienyl-2-Rest oder einen aromatischen Rest der Formel darstellt:

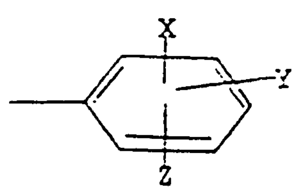

worin X, Y, Z, gleich oder verschieden, ein Wasserstoffatom, einen Niedrigalkylrest, einen Trifluormethylrest, einen Niedrigalkoxyrest, ein Halogenatom, eine Nitro- oder Hydroxylgruppe darstellen;

- Triacetoxy-1,8,9-anthracen der Formel:

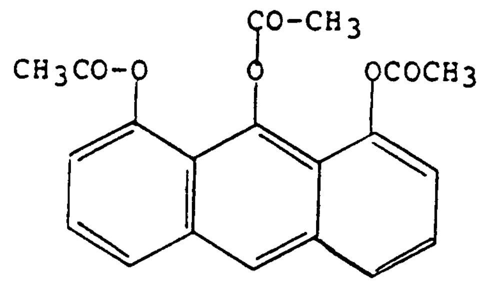

- den Mono-, Di- und Triestern von Dihydroxy-1,8-phenyl-10-anthron-9 oder -anthranol-9 der folgenden allgemeinen Formel:

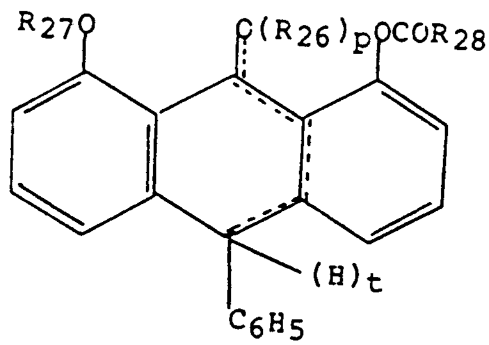

worin: p 0 oder 1 ist;

. wenn p = 0, t = 1, bedeutet $R_{27}$ ein Wasserstoffatom oder $-COR_3$;

. wenn P = 1, t = 0, bedeuten $R_{26}$ und $R_{27}$, unabhängig voneinander, ein Wasserstoffatom oder $-COR_{28}$;

wobei $R_{28}$ einen linearen oder verzweigten Alkylrest mit 1 bis 17 Kohlenstoffatomen, einen Cycloalkylrest oder einen Phenylrest darstellt, der gegebenenfalls durch einen Niedrigalkylrest, einen Niedrigalkoxyrest, ein Halogenatom, eine Nitrofuktion, einen $-CF_3$-Rest oder durch eine Hydroxylfunktion substituiert ist,

sowie den Mischungen dieser Ester.

**3.** Assoziat gemäß Anspruch 1,
dadurch **gekennzeichnet,** daß
das Pyridinderivat der Formel (I) unter den Verbindungen ausgewählt ist, in denen $R_{29}$ Wasserstoff und $R_1$ eine

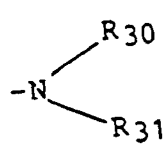

-Gruppe darstellen, worin $R_{30}$ und $R_{31}$ einen Piperidinylring bilden.

**4.** Assoziat gemäß jedem Anspruch 1 bis 3,
dadurch **gekennzeichnet,** daß

die Verbindung der Formel (I) aus Amino-6-dihydro-1,2-hydroxy-1-imino-2-piperidino-4-pyrimidin oder "Minoxidil" zusammengesetzt ist.

**5.** Assoziat gemäß jedem Anspruch 1 bis 3, dadurch **gekennzeichnet,** daß das Derivat von 1,8-Hydroxy- und/oder -acyloxyanthracen oder -anthron aus 1,8,9-Trihydroxyanthracen oder 1,8-Dihydroxy-9-anthron oder "Anthralin" zusammengesetzt ist.

**6.** Assoziat gemäß jedem Anspruch 1 bis 4, dadurch **gekennzeichnet,** daß der Bestandteil (A) das Derivat von 1,8-Hydroxy- und/oder -acyloxyanthracen oder -anthron in Mengen von 0,01 bis 10 Gew.%, bevorzugt von 0,1 bis 1 Gew.%, noch bevorzugter von 0,1 bis 0,5 Gew.%, bezogen auf das Gewicht des Bestandteils (A), enthält.

**7.** Assoziat gemäß jedem Anspruch 1 bis 5, dadurch **gekennzeichnet,** daß der Bestandteil (B) die Verbindung der Formel (I) in Mengen von 0,05 bis 10 Gew.%, vorzugsweise von 0,05 bis 5%, und insbesondere von 0,5 bis 4 %, bezogen auf das Gewicht der Gesamtzusammensetzung, enthält.

**8.** Assoziat gemäß jedem Anspruch 1 bis 6, dadurch **gekennzeichnet,** daß das Gewichtsverhältnis von Pyrimidinderivat der Formel (I) zu 1,8-Hydroxy- und/oder -acyloxyanthracen oder -anthron von 2/1 bis 10/1 ausmacht.

**9.** Assoziat gemäß jedem Anspruch 1 bis 8, dadurch **gekennzeichnet,** daß es in Form einer einzigen Zusammensetzung vorliegt, enthaltend die Bestandteile (A) und (B), worin das Derivat von 1,8-Hydroxy- und/oder -acyloxyanthracen oder -anthron in Mengen von vorzugsweise 0,01 bis 5 Gew.%, bezogen auf Gesamtgewicht der Zusammensetzung, und das Pyrimidinderivat der Formel (I) in Mengen von 0,05 bis 6 Gew.%, bezogen auf Gesamtgewicht der Zusammensetzung, vorliegen.

**10.** Assoziat gemäß jeden Anspruch 1 bis 9, dadurch **gekennzeichnet,** daß die physiologisch verträglichen Medien aus einem oder mehreren wasserfreien Lösungsmitteln oder einer Mischung aus Wasser und einem oder mehreren Lösungsmittel(n) zusammengesetzt sind, wobei die Lösungsmittel unter Niedrigalkoholen, Alkylenglycolen oder Alkylethern von Alkylenglycolen oder Dialkylenglycolen ausgewählt sind.

**11.** Assoziat gemäß jedem Anspruch 1 bis 10, dadurch **gekennzeichnet,** daß mindestens eines der physiologisch verträglichen Medien durch Verdickungs- und/oder Geliermittel verdickt ist.

**12.** Assoziat gemäß jedem Anspruch 1 bis 11, dadurch **gekennzeichnet,** daß mindestens einer der Bestandteile (A) oder (B) auch einen kosmetisch oder pharmazeutisch verträglichen Hilfsstoff enthält, ausgewählt unter Konservierungsmitteln, Komplexiermitteln, Färbemitteln, alkalisch oder sauer machenden Mitteln, anionischen, kationischen, nicht-ionischen oder amphoteren oberflächenaktiven Mitteln sowie deren Mischungen, anionischen, kationischen, nicht-ionischen oder amphoteren Polymeren sowie deren Mischungen.

**13.** Verfahren zur kosmetischen Behandlung der Haare oder der beharten Haut, dadurch **gekennzeichnet,** daß man die Bestandteile (A) und (B) des Assoziats gemäß jedem Anspruch 1 bis 12 getrennt aufbringt, sei es gleichzeitig, sei es aufeinanderfolgend oder nach einem Zeitablauf.

14. Vorrichtung aus mehreren Teilen oder "Kit",
dadurch **gekennzeichnet,** daß
sie in einem ersten Teil einen Bestandteil (A), enthaltend in einem physiologisch verträglichen Milieu ein Derivat von 1,8-Hydroxy- und/oder -acyloxyanthracen oder -antrhon gemäß der in Anspruch 1 oder 2 gegebenen Definition, und in einem zweiten Teil einen Bestandteil (B), enthaltend in einem physiologisch verträglichen Milieu ein Pyrimidinderivat gemäß Formel (I), umfassen.

15. Assoziat gemäß jedem Anspruch 1 bis 12 zur therapeutischen Anwendung in der Behandlung des Haarausfalls.

16. Verwendung des Assoziats gemäß jedem Anspruch 1 bis 12 zur Herstellung eines Medikaments zur Anwendung in der therapeutischen Behandlung des Haarausfalls.